Europäisches Patentamt

European Patent Office

Office européen des brevets

(19)

(11) Veröffentlichungsnummer: **0 302 053**
**B1**

(12) **EUROPÄISCHE PATENTSCHRIFT**

(45) Veröffentlichungstag der Patentschrift: **31.10.90**

(51) Int. Cl.⁵: **A 61 M 1/00,** A 61 M 16/04

(21) Anmeldenummer: **86904094.9**

(22) Anmeldetag: **10.07.86**

(86) Internationale Anmeldenummer:
**PCT/DE86/00283**

(87) Internationale Veröffentlichungsnummer:
**WO 87/05522 24.09.87 Gazette 87/21**

(54) ROHRFÖRMIGE BIEGSAME SONDE ZUM EINFÜHREN IN DIE LUFTRÖHRE BZW. IN DAS BRONCHIALSYSTEM.

(30) Priorität: **18.03.86 DE 3608943**

(43) Veröffentlichungstag der Anmeldung:
**08.02.89 Patentblatt 89/06**

(45) Bekanntmachung des Hinweises auf die Patenterteilung:
**31.10.90 Patentblatt 90/44**

(84) Benannte Vertragsstaaten:
**AT BE CH DE FR GB IT LI LU NL SE**

(56) Entgegenhaltungen:
**DE-A-1 491 652**
**DE-B-2 364 119**
**FR-A-2 454 308**
**US-A-4 300 550**
**US-A-4 468 216**

(73) Patentinhaber: **Schmidt, Christoph**
**Am Kümpel 12**
**D-5300 Bonn 1 (DE)**
(73) Patentinhaber: **Schön, Rudolf**
**Im Spichelsfeld 34**
**D-5202 St. Augustin (DE)**
(73) Patentinhaber: **Russ, Jürgen**
**Rosental 32**
**D-5300 Bonn 1 (DE)**

(72) Erfinder: **Schmidt, Christoph**
**Am Kümpel 12**
**D-5300 Bonn 1 (DE)**
Erfinder: **Schön, Rudolf**
**Im Spichelsfeld 34**
**D-5202 St. Augustin (DE)**
Erfinder: **Russ, Jürgen**
**Rosental 32**
**D-5300 Bonn 1 (DE)**

(74) Vertreter: **Müller-Gerbes, Margot**
**Friedrich-Breuer-Strasse 112**
**D-5300 Bonn 3 (Beuel) (DE)**

Courier Press, Leamington Spa, England.

**Beschreibung**

Die Erfindung betrifft eine rohrförmige biegsame Sonde zum Einführen in die Luftröhre bzw. in das Bronchialsystem mit einem von einem Rohr, das einen Außehdurchmesser von etwa 5 bis 8 mm aufweist, gebildeten durchgängige Kanal, dessen Ausgang am Rohrende mündet.

Sonden der gattungsgemäßen Art, wie beispielsweise aus der DE-AS 23 64 119 bekannt, werden für die Absaugung von Schleim und Wasser aus der Lunge und Luftröhre verwendet. Der rohrförmige biegsame Katheter weist dabei einen Außendurchmesser von etwa 5 bis 8 mm bei einem Innendurchmesser für den Absaugkanal von etwa 3,5 bis 6 mm bei Wanddicken von 0,71 bis 0,812 mm auf. Die am in das Bronchialsystem eingeführten Ende ausgebildete Ringwulst erleichtert das Einführen der Sonde und verhindert das Festsaugen sowie Verletzungen an der Schleimhaut und den Bronchien. Solche Katheter sind für den einmaligen kurzzeitigen Gebrauch bestimmt.

In neuerer Zeit ist man bemüht, bei der Behandlung von Notfällen Medikamente direkt in die Lunge des betroffenen Menschen einzubringen. Wenn man hierfür die bekannten Absaugkatheter z.B. gemäß DE-AS 23 64 119 benutzt, so hat das den großen Nachteil, daß die Medikamente nach dem Absaugen durch den Absaugkanal eingebracht werden müssen, und infolge der Größe dieses Kanals nur zu geringen Anteilen bis in die Lunge gelangen, im übrigen jedoch im Katheterrohr verbleiben. Es gelingt nicht, die Medikamente vollständig und tief in die Lunge einzubringen und zu verteilen. Auch sind die bekannten Katheter nicht mit Ansatzstücken zum Ansetzen einer Spritze ausgerüstet.

Zur Beatmung und Sauerstoffzufuhr sind für den Dauergebrauch sogenannte Beatmungstuben oder Ballonkatheter, siehe DE-OS 23 08 400 oder US-PS 43 00 550 u.a. bekannt, die relativ kurz sind und nur in die Trachea eingeführt werden. Sie sind zum Einführen in das Bronchialsystem wegen ihrer Kürze und wegen ihres offenen, meist abgeschrägten Endes, das zu erheblichen Verletzungen des Bronchialgewebes führen würde, ungeeignet. Essind auch doppellumige Wunddrainage-Katheter z.B. aus der US-PS 44 68 216 oder FR-A 24 54 308 bekannt, die intraoperativ von innen nach außen unter Sichtkontrolle gelegt werden. Diese Wunddrainage-Katheter enthalten innerhalb eines großen Absaugkanals für die Wundflüssigkeit koaxial einen Schlauch mit sehr kleinen Querschnittabmessungen zum Einführen von Spülflüssigkeit. Um das Absaugen nicht zu behindern, muß dieser innenliegende Schlauch einen sehr kleinen Außendurchmesser aufweisen, so daß er keine Eigenfestigkeit mehr aufweist und für sich allein nicht in eine Wunde oder Luftröhre einführbar ist, da er abknicken würde.

Der Erfindung liegt die Aufgabe zugrunde, eine Sonde bzw. einen Katheter zu schaffen, mit der Medikamente durch die Luftröhre bzw. Bronchientief in das Bronchialsystem bzw. die Lunge eingebracht werden können.

Die Erfindung löst die gestellte Aufgabe mit einer rohrförmigen biegsamen Sonde, die zum Einführen in die Luftröhre bzw. das Bronchialsystem geeignet ist, dadurch, daß das Rohr eine Länge von etwa 30 bis 50 cm aufweist, daß der Kanal einen Durchmesser von etwa 1 mm bis zu maximal etwa 2 mm zum Durchleiten von Medikamenten und/oder Spüllösung mit hoher Strömungsgeschwindigkeit aufweist und die Wanddicke des Rohres mindestens 1,5 mm beträgt und das Eingangsende mit einem Ansatzstück zum Ansetzen einer Spritze ausrüstbar ist.

Der Erfindung liegt die Erkenntnis zugrunde, daß eine möglichst, hohe Druckdifferenz zwischen den Enden der Sonde notwendig ist, um damit eine hohe Strömungsgeschwindigkeit zum tiefen Einbringen und Verteilen von Medikamenten in die Lunge zu erzielen. Hierfür sind sehr kleine Kanalquerschnitte im Sinn der Erfindung erforderlich. Ein Schlauch mit einem so kleinen Kanaldurchmesser weist bei normaler Ausführung der Wanddicke einen nicht viel größeren Außendurchmesser auf und ist daher so biegsam, daß er sich nicht mehr einwandfrei in das Bronchialsystem einführen läßt.

Die Erfindung baut auf einer zum Einführen in das Bronchialsystem geeigneten Sonde mit Außenabmessungen in einer Größe und Ausgestaltung, wie sie zum Absaugen von Schleim und Wasser aus der Lunge bekannt ist, auf und bildet eine solche Sonde erfindungsgemäß mit einem Kanal mit so kleinem Querschnitt aus, der eine hohe Druckdifferenz zum tiefen Einbringen von Medikamenten in die Lunge mit hoher Strömungsgeschwindigkeit ermöglicht. Gleichzeitig wird der Eingang dieses Medikamentenkanals mit einem Ansatzstück zum Ansetzen einer Sprizte ausgerüstet, wobei das Ansatzstück als separates Bauteil oder integriert mit dem Rohr bzw. Kanal ausgebildet sein kann.

Die Sonde ist erfindungsgemäß so gestaltet, daß sie nur die Einleitung von Medikamenten mittels eines einzigen vorhandenen kleinen Kanals — Medikamentenkanal oder Applikationskanal — mit hoher Strömungsgeschwindigkeit erlaubt. Die Größe des Rohres sorgt für die einwandfreie Einführung in das Bronchialsystem. Das Ansatzstück für die Spritze kann direkt am Rohrende angeformt sein — integriert ausgebildet mit durchgehendem Kanal bzw. Bohrung. Das Rohr zeichnet sich durch eine relativ große Wanddicke von mindestens etwa 1,5 mm aus. Hierfür kommen dann nur sehr biegsame, weiche, flexible Kunststoffe infrage. Bei diesen erfindungsgemäß ausgebildeten Sonden kann das in die Luftröhre einzuführende Rohrende auch abgerundet verjüngt ausgebildet werden anstelle einer Ringwulst. Insbesondere ist es auch möglich, den Ausgang des kleinen Applikationskanals auf der Mantelfläche des Rohres im Bereich des Rohrendes anzuordnen. Auch kann der Ausgang des Kanals düsenförmig erweitert sein.

Das Rohr weist bei allen Sonden eine Länge von etwa 30 bis 50 cm auf, es ist aus einem biegsamen flexiblen und sterilierbaren Material, beispielsweise Kunststoff hergestellt. Das Rohr kann hierbei einen Außendurchmesser von etwa 5 bis 8 mm aufweisen, wobei der Kanal für die Einführung der Medikamente in die Lungen einen lichten Durchmesser von etwa 1 bis 1,5 mm maximal bis 2 mm aufweisen sollte.

Die Ansatzstücke für die Spritzen werden vorteilhaft mit einer Verschließeinrichtung, wie Ventil, Stöpsel, Verschlußklappe ausgerüstet. Des weiteren ist es nach einem weiteren Vorschlag der Erfindung möglich, die Rohrwandung in axialer Längserstreckung mit einem Röntgenkontraststreifen auszurüsten. Somit ist eine Röntgenkontrolle während der Behandlung möglich.

Mit der Erfindung wird auf dem in Rede stehenden medizinischen Gebiet ein erheblicher Fortschritt bei der Behandlung von Patienten erreicht. Bisher war es nur möglich, bei der Reanimation mit den vorhandenen Geräten in der folgenden Reihenfolge zu arbeiten:

1) Einführen eines Beatmungstubus in die Trachea

2) Einbringen von Spüllösung in den Beatmungstubus

3) Ventilation mit einem Beatmungsbeutel zur Verteilung der Spülflüssigkeit

4) Einführen eines Saugkatheters mit ausschließlicher Saugung

5) Entfernen des Saugkatheters

6) Einführung eines Cava -Katheters, wobei die Gefahren der Schleimhautverletzung gegeben sind (behelfsmäßige Lösung)

7) Einbringen von Notfallmedikamenten über den Cava -Katheter

8) Entfernen des Cava -Katheters

9) Einsetzen der volumenkontrollierten Beatmung.

Der Zeitaufwand hierfür beträgt insgesamt etwa 3 bis 3 ½ Minuten.

Bei dieser Therapie ist eine Schädigung durch mangelnde Sauerstoffversorgung des Notfallpatienten nicht auszuschließen. Auch ist eine ausreichende Applizierung der Notfallmedikamente durch den Cava -Katheter nicht immer gewährleistet. Hinzu kommt der hohe Materialverbrauch, der arbeitsaufwendig und unwirtschaftlich ist.

Mit der Erfindung wird es möglich, den Notfallpatienten durch Einführen der erfindungsgemäß ausgebildeten Sonde über die Luftröhre und tiefe Applikation von Notfallmedikamenten mit der Sonde bis in die Lunge zu versorgen.

Durch Anwendung der Erfindung wird eine wesentlich schnellere Behandlung des Notfallpatienten ermöglicht, die seine Oberlebenschancen erheblich steigern. Eine Schädigung des zentralen Nervensystems kann auf ein äußerstes Minimum reduziert werden. Darüber hinaus ist der zu betreibende Arbeitsaufwand wesentlich geringer und auch der Materialeinsatz.

Die Erfindung wird in der Zeichnung an Beispielen erläutert, ist jedoch nicht auf diese beschränkt.

Es zeigen in schematischer Darstellung

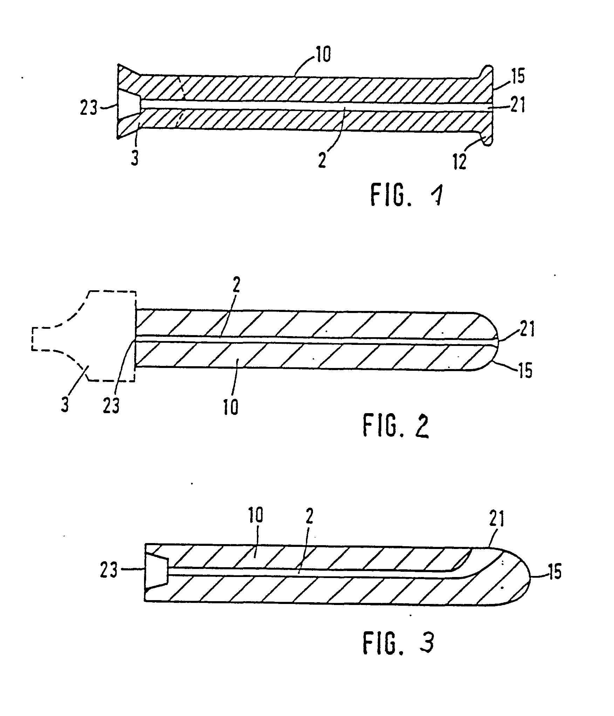

Figur 1—3 Längsschnitt verschiedener Sonden mit je einem Kanal zu Applikation von Medikamenten.

Die Sonde zum Einführen in das Bronchialsystem, die nur zum direkten Einführen von Medikamenten in die Lunge ausgebildet ist, enthält das äußere Rohr 10. Das Rohr 10 ist beispielsweise aus einem inerten Kunststoffschlauch hergestellt und biegsam.

In den Figuren 1 bis 3 sind verschiedene Ausführungen von erfindungsgemäßen Sonden mit einem Lumen, die als Katheter für die intrabronchiale Applikation von Medikamenten verwendet werden, dargestellt.

Bei der Sonde gemäß Fig. 1 ist das Ansatzstück 3 für die Spritze integriert mit dem dickwandigen Rohr 10 ausgebildet und am Rohrausgang 15 die Ringwulst 12 ausgebildet. Der kleine Medikamentenkanal 2 läuft axial durch den Eingang 23 bis zum Ausgang 21 am Rohrende.

Bei der Sonde gemäß Figur 2 ist das Rohrende 15 verjüngt und der Kanalausgang 21 des Kanals 2 zentral an der Spitze, ggf. leicht düsenförmig erweitert, ausgebildet. Das Ansatzstück 3 für die Spritze ist aufgesetzt.

Bei der Sonde gemäß Figur 3 ist das Rohrende 15 ebenfalls abgerundet verjüngt, der Ausgang 21 des Kanals 2 jedoch seitlich an der Mantelfläche des Rohres 10 im Bereich des Rohrendes vorgesehen.

## Patentansprüche

1. Rohrförmige biegsame Sonde zum Einführen in die Luftröhre bzw. in das Bronchialsystem mit einem von einem Rohr, das einen Außendurchmesser von etwa 5 bis 8 mm aufweist, gebildeten durchgängigen Kanal, dessen Ausgang am Rohrende mündet, dadurch gekennzeichnet, daß das Rohr eine Länge von etwa 30 bis 50 cm aufweist, daß der Kanal (2) einen Durchmesser von etwa 1 mm bis zu maximal etwa 2 mm zum Durchleiten von Medikamenten und/oder Spüllösung mit hoher Strömungsgeschwindigkeit aufweist und die Wanddicke des Rohres (10) mindestens 1,5 mm beträgt und das Eingangsende (23) mit einem Ansatzstück (3) zum Ansetzen einer Spritze ausrüstbar ist.

2. Sonde nach Anspruch 1, dadurch gekennzeichnet, daß der Kanal (2) einen Durchmesser von etwa 1 bis 1,5 mm hat.

3. Sonde nach Anspruch 1 oder 2, dadurch gekennzeichnet, da das Ansatzstück (3) für die Spritze mit dem Eingangsende (23) des Kanals (2) integriert ausgebildet und am Rohrende angeformt ist.

4. Sonde nach einem der Ansprüche 1 bis 3, dadurch gekennzeichnet, daß das Ausgangsende (15) des Rohres (10) abgerundet verjüngt ausgebildet ist.

5. Sonde nach Anspruch 4, dadurch gekennzeichnet, daß der Ausgang (21) des Kanals (2) sich auf der Mantelfläche des Rohres (10) befindet.

6. Sonde nach einem der Ansprüche 1 bis 3, dadurch gekennzeichnet, daß das Ausgangsende (15) außenseitig von einem Ringwulst umgeben ist.

7. Sonde nach einem der Ansprüche 1 bis 6, dadurch gekennzeichnet, daß in axialer Längserstreckung die Wandung der Sonde (10) mit einem Röntgenkonstraststreifen ausgerüstet ist.

8. Sonde nach einem der Ansprüche 1 bis 7, dadurch gekennzeichnet, daß der Ausgang (21) des Kanals düsenförmig erweitert ist.

9. Sonde nach einem der Ansprüche 1 bis 8, dadurch gekennzeichnet, daß das Ansatzstück mit einer Verschließeinrichtung, wie Stöpsel, Ventil, Verschlußkapsel ausgerüstet ist.

**Revendications**

1. Sonde tubulaire flexible à introduire dans la trachée ou dans le système bronchique, avec un conduit continu formé par un tube présentant un diamètre extérieur d'environ 5 à 8 mm, dont la sortie débouche à l'extrémité du tube, caractérisée en ce que le tube présente une longueur d'environ 30 à 50 cm, que le conduit (2) présente un diamètre d'environ 1 mm à environ 2 mm au maximum pour le passage de médicaments et/ou de solution de rinçage à grande vitesse d'écoulement, et que l'épaisseur de paroi du tube (10) est d'au moins 1,5 mm et que l'extrémité d'entrée (23) peut être pourvue d'un embout (3) pour une seringue.

2. Sonde selon la revendication 1, caractérisée en ce que le conduit (2) présente un diamètre d'environ 1 à 1,5 mm.

3. Sonde selon la revendication 1 ou 2, caractérisée en ce que l'embout (3) pour la seringue est formé en étant intégré avec l'extrémité d'entrée (23) du conduit (2) et est moulé sur l'extrémité du tube.

4. Sonde selon l'une des revendications 1 à 3, caractérisée en ce que l'extrémité de sortie (15) du tube (10) est de configuration s'effilant en arrondi.

5. Sonde selon la revendication 4, caractérisée en ce que la sortie (21) du conduit (2) se trouve sur la surface de l'enveloppe du tube (10).

6. Sonde selon l'une des revendications 1 à 3, caractérisée en ce que l'extrémité de sortie (15) est entourée extérieurement d'un bourrelet annulaire.

7. Sonde selon l'une des revendications 1 à 6, caractérisée en ce que la paroi de la sonde (10) est pourvue d'une bande de contraste aux rayons X s'étendant longitudinalement dans le sens axial.

8. Sonde selon l'une des revendications 1 à 7, caractérisée en ce que la sortie (21) du conduit s'élargit en forme de tubulure.

9. Sonde selon l'une des revendications 1 à 8, caractérisée en ce que l'embout est équipé d'un dispositif de fermeture tel qu'un bouchon, une soupape, une capsule de fermeture.

**Claims**

1. Tubular flexible probe for insertion into the wind-pipe or into the bronchial system with a general duct formed by a tube which has an outer diameter of about 5 to 8 mm, the output of the duct opening out at the end of the tube, characterized in that the tube has a length of about 30 to 50 cm, in that the duct (2) has a diameter of about 1 mm to a maximum of about 2 mm for passing through medicaments and/or rinsing solution at high flow speed and the wall thickness of the tube (10) amounts to at least 1.5 mm and the input end (23) can be equipped with an attachment piece (3) for attaching a spray.

2. Probe according to claim 1 characterized in that the duct (2) has a diameter of about 1 to 1.5 mm.

3. Probe according to claim 1 or 2, characterized in that the attachment piece (3) for the spray is constructed in an integrated manner with the input end (23) of the duct (2) and is integrally moulded at the end of the tube.

4. Probe according to one of claims 1 to 3, characterized in that the output end (15) of the tube (10) is constructed in a rounded off and tapered manner.

5. Probe according to claim 4, characterized in that the output (21) of the duct (2) is located on the casing surface of the tube (10).

6. Probe according to one of claims 1 to 3, characterized in that the output end (15) is surrounded on the outside by an annular ring.

7. Probe according to one of claims 1 to 6, characterized in that, in the axial longitudinal extension, the wall of the probe (10) is equipped with an X-ray contrast strip.

8. Probe according to one of claims 1 to 7, characterized in that the output (21) of the duct is widened in the shape of a nozzle.

9. Probe according to one of claims 1 to 8, characterized in that the attachment piece is equipped with a closing device, such as stopper, valve, closing cap.

FIG. 1

FIG. 2

FIG. 3